(19) Europäisches Patentamt
European Patent Office
Office européen des brevets

(11) **EP 0 794 729 B1**

(12) **EUROPÄISCHE PATENTSCHRIFT**

(45) Veröffentlichungstag und Bekanntmachung des Hinweises auf die Patenterteilung:
**11.04.2001 Patentblatt 2001/15**

(51) Int Cl.[7]: **A61B 5/0275**

(21) Anmeldenummer: **95940114.2**

(22) Anmeldetag: **29.11.1995**

(86) Internationale Anmeldenummer:
**PCT/DE95/01690**

(87) Internationale Veröffentlichungsnummer:
**WO 96/16594 (06.06.1996 Gazette 1996/26)**

(54) **VORRICHTUNG ZUR ERMITTLUNG DER HIRNDURCHBLUTUNG UND DES INTRACRANIELLEN BLUTVOLUMENS**

DEVICE FOR DETERMINING CEREBRAL BLOOD FLOW AND INTRACEREBRAL BLOOD VOLUME

DISPOSITIF DE DETERMINATION DU DEBIT SANGUIN CEREBRAL ET DU VOLUME SANGUIN INTRACEREBRAL

(84) Benannte Vertragsstaaten:
**DE FR GB IT**

(30) Priorität: **01.12.1994 DE 4442751**

(43) Veröffentlichungstag der Anmeldung:
**17.09.1997 Patentblatt 1997/38**

(73) Patentinhaber: **Hoeft, Andreas, Prof. Dr. med.**
**53127 Bonn (DE)**

(72) Erfinder: **Hoeft, Andreas, Prof. Dr. med.**
**53127 Bonn (DE)**

(74) Vertreter: **Körner, Peter**
**Thömen & Körner**
**Zeppelinstrasse 5**
**30175 Hannover (DE)**

(56) Entgegenhaltungen:
• **IEEE PROCEEDINGS OF THE 23RD ANNUAL CONFERENCE ON ENGINEERING IN MEDICINE AND BIOLOGY, Bd. 12, 16.November 1970, NEW YORK, US, Seite 276 XP002002985 WILSON ET AL.: "Measurment of jugular venous flow by thermal dilution"**
• **BIOMEDIZINISCHE TECHNIK, Bd. 28, Nr. 10, Oktober 1983, BERLIN, DE, Seiten 216-220, XP002002986 HOEFT ET AL.: "Messung der Koronardurchblutung mit einem Doppelfiberoptiksystem und rechnergestützter Entfaltung von transkoronaren Farbstoffdilutionssignalen"**
• **JOURNAL OF APPLIED PHYSIOLOGY, Bd. 64, Nr. 3, März 1988, US, Seiten 1210-1216, XP000570031 BÖCK ET AL.: "Thermal recovery after passage of pulmonary circulation assessed by deconvolution"**
• **PHYSICS IN MEDICINE AND BIOLOGY, Bd. 37, Nr. 11, November 1992, BRISTOL, GB, Seiten 2059-2069, XP000309567 SCHRÖDER ET AL.: "Calculation of body transport function"**

**Beschreibung**

Eine jederzeit ausreichende Durchblutung des Gehirns ist nicht nur eine unabdingbare Voraussetzung für eine normale Hirnfunktion, sondern auch eine essentielle Notwendigkeit für ein Überleben des Individuums in Situation mit eingeschränkter Herzkreislauffunktion. Bereits kurzfristige Unterbrechungen der Hirndurchblutung können zu irreversiblen Schäden führen, die mit dem Leben nicht vereinbar sind oder mit schwersten Defektheilungen einhergehen.

In der Medizin stellt die Überwachung der Hirndurchblutung ein besonderes Problem bei Patienten ohne Bewußtsein dar, z.B. während einer Narkose oder bei beatmeten Intensivpatienten. Bei diesen Patienten muß die Hirnfunktion auch zur Schmerzausschaltung medikamentös unterdrückt werden und somit besteht keine Möglichkeit indirekt zu beurteilen, in wieweit die Durchblutung des Gehirns ausreichend ist. Eine besonders schwierige Problematik besteht bei Patienten mit Erkrankungen des Gehirns, wie z.B. schwersten Gehirnerschütterungen und Gehirntumoren.

Einerseits neigt das Gehirn in diesen Fällen zum Anschwellen, wodurch die Durchblutung behindert werden kann. Andererseits kann es auch zur Entkopplung der Hirndurchblutung vom Stoffwechselbedarf des Gehirns kommen, wobei die begleitende Weitstellung der Blutgefäße im Gehirn ihrerseits eine Hirnschwellung verursachen kann. Während in dem einen Fall therapeutische Maßnahmen ergriffen werden müßten, die auf Förderung der Hirndurchblutung abzielen, müßte in dem anderen Fall eher eine Verminderung der Hirndurchblutung angestrebt werden.

Eine einfache, vor allem auch am Krankenbett anwendbare Methode, zur Messung der Hirndurchblutung und des intracraniellen Blutvolumens erscheint daher ebenso wünschenswert, wie eine einfache Kontrollmöglichkeit bei der Prüfung neu entwickelter Medikamente zur Förderung oder Verminderung der Hirndurchblutung auf Wirksamkeit und Dosierung.

Bislang stehen nur relativ aufwendige Verfahren zur Verfügung um die Hindurchblutung am Patienten zu messen. In der Regel handelt es sich um Indikatorverdünnungsverfahren, bei denen Fremdgase als Indikatoren verwendet werden. Bei einer seit langem gebräuchlichen Technik (Kety-Schmid Verfahren) wird eine Aufsättigung des Blutes und des Gehirns mit Lachgas oder Argon durchgeführt, wobei das betreffende Gas in der Atemluft angereichert wird. Die Analytik erfolgt mittels mehrerer Blutproben zeitgleich im arteriellen und im zerebralvenösen Blut, wozu erstens ein arterieller Katheter und zweitens ein Katheter im "bulbus venae jugularis" benötigt wird. Letzterer wird über eine Halsvene (vena jugularis interna) retrograd bis auf die Höhe der Schädelbasis eingeführt.

Bei einer anderen Methodik wird radioaktiv markiertes Xenon verwendet, das als Bolus in wäßriger Lösung injiziert wird. Bei dieser Technik erfolgt der Gasnachweis mittels extracranieller Detektoren. Beide Methoden sind während Operationen oder bei Intensivpatienten wegen der hiermit verbundene Kosten sowie wegen der erforderlichen Ausrüstung nur zu wissenschaftlichen Zwecken durchführbar und für die klinische Routine ungeeignet. Bei dem Kety-Schmid Verfahren müssen die Blutproben mit einer aufwendigen Laboranalytik aufgearbeitet werden, so daß das Ergebnis meist erst nach Stunden zur Verfügung steht. Die Xenon Technik weist neben den hohen Kosten vor allem den Nachteil der Strahlenbelastung für Patienten und für Personal auf.

Aus Journal of Applied Physiology, Bd. 64, Nr. 3, März 1988, Seiten 1210-1216, Böck et al.: "Thermal recovery after passage of pulmonary circulation assessed by deconvolution" ist es bekannt, das Herzzeitvolumen und das extravasculäre thermische Lungenvolumen zu bestimmen. Hierzu wird ein Doppelindikator aus Wärme/Kälte einerseits und Farbstoff andererseits, dessen Menge genau bekannt sein muß, in die Blutbahn injiziert. Danach werden die Indikatorverdünnungskurven gemessen und anschließend die Flächen unter den Kurven bestimmt. Das Verhältnis der Menge des Indikators zur Fläche unter der Kurve ergibt dann den Blutfluß in absoluten Einheiten, z.B. in Milliliter pro Minute.

Mit dem Indikator Wärme/Kälte wird der Blutfluß "Herzzeitvolumen" bestimmt. Das extravasculäre thermische Lungenvolumen wird aus dem Produkt von Herzzeitvolumen und mittlerer Transitzeit bestimmt, wobei hierzu das intravasculäre Lungenvolumen abgezogen wird, in dem die Differenz zwischen der mittleren Transitzeit des Indikators Wärme/Kälte und des Indikators Farbstoff gebildet wird.

Der Erfindung liegt die Aufgabe zugrunde, eine Vorrichtung zur Ermittlung der Hirndurchblutung und des intracraniellen Blutvolumens zu schaffen, die bei geringstmöglicher körperlicher Beeinträchtigung eine einfache, schnelle und präzise Ermittlung dieser Größen ermöglicht.

Diese Aufgabe wird durch eine Vorrichtung mit den Merkmalen des Anspruchs 1 gelöst. Weiterbildungen und vorteilhafte Ausgestaltungen ergeben sich aus den Unteransprüchen.

Die erfindungsgemäße Vorrichtung nutzt ein Doppelindikatorverdünnungsverfahren mit einer in ihrer Injektionstemperatur von der Körpertemperatur abweichende Farbstofflösung, bei der Wärme oder Kälte eine hochdiffusible Indikatorkomponente und Farbstoff, vorzugsweise Indocyaningrün ICG, eine intravasale Indikatorkomponente bilden. Aus den unterschiedlichen Transportprozessen der beiden Indikatorkomponenten ergibt sich die Möglichkeit, neben der Hirndurchblutung auch das intracranielle Blutvolumen zu bestimmen.

Die Hirndurchblutung CBF = "cerebral blood flow" wird aus der mittleren Transitzeit der Wärme oder Kälte durch das Gehirn $mtt_{therm}$ ermittelt, wobei folgender Zusammenhang gilt:

$$(1) \qquad CBF = \lambda_{therm} / mtt_{therm}$$

Der Faktor $\lambda_{therm}$ ist hierbei der Verteilungskoeffizient des Indikators Wärme oder Kälte zwischen Gehirngewebe und Blut. Er stellt eine Naturkonstante dar, die sich entweder durch einmalige Vergleichsmessungen mit anderen Verfahren zur Bestimmung der Hirndurchblutung empirisch ermitteln oder über die spezifische Wärmekapazität der Gehirnsubstanz berechnen läßt.

Das intracranielle Blutvolumen ICBV = "intracerebral blood volume" berechnet sich aus dem Produkt des CBF und der mittleren Transitzeit des intravasalen Farbstoffindikators ICG $mtt_{ICG}$:

$$(2) \qquad ICBV = CBF \cdot mtt_{ICG}$$

Die Einbringung des Doppelindikators erfolgt durch Bolusinjektion, vorzugsweise zentralvenös über einen entsprechenden Katheter. Weiterhin erfolgt eine Messung der arteriellen und zerebralvenösen Indikatorverdünnungskurven. Dies kann mit Hilfe von kombinierten Fiberoptik-Thermistor-Kathetern durchgeführt werden, die über die arteria femoralis in der Aorta und über die vena jugularis interna im bulbus venae jugularis plaziert werden.

Die mittleren Transitzeiten $mtt_{ICG}$, $mtt_{therm}$ können aus den transcerebralen Transportfunktionen der beiden Indikatorkomponenten $g_{therm}(t)$ bzw. $g_{IGC}(t)$ bestimmt werden. Für die Transportfunktionen g(t), die arterielle a(t) und cerebralvenöse v(t) Indikatorkurve gilt allgemein ein Zusammenhang, der durch das folgende Faltungsintegral ausgedrückt wird:

$$(3) \qquad v(t) \;=\; \int_{0}^{\infty} g(t-u)a(u)\,du$$

Die mittlere Transitzeit entspricht dem ersten Moment der Transportfunktion:

$$(4) \qquad mtt \;=\; \frac{\int_{0}^{\infty} g(t)\cdot t \; dt}{\int_{0}^{\infty} g(t)\,dt}$$

Liegen, wie in diesem Fall, die arteriellen und die venösen Indikatorverdünnungskurven als Meßsignale vor, so müssen die Transportfunktionen durch "Entfaltung" von a(t) und v(t) ermittelt werden. Dies kann durch modellfreie Verfahren durchgeführt werden. Numerisch günstiger ist jedoch die Verwendung von Modellfunktionen. Ist die grundsätzliche Form der Transportfunktionen bekannt und durch eine Funktion, z.B. durch eine Lognormalverteilung, beschreibbar, so können für gemessene Kurvenpaare von a(t) und v(t) die Parameter der zugehörigen Transportfunktionen, insbesondere die mittleren Transitzeiten, durch ein iteratives nichtlineares Anpassungsverfahren ermittelt werden. Hierbei wird nach dem Verfahren der kleinsten Quadrate unter iterativer Variation der Parameter der Transportfunktion g(t) die Differenz zwischen gemessener cerebralvenöser Konzentration und dem Faltungsergebnis der arteriellen Kurve mit der Transportfunktion minimiert:

$$(5) \qquad \left[ v(t) - \int_{0}^{\infty} g(t-u)\,a(u)\,du \right]^{2} \;=\; \min$$

Zur Beschreibung von intravasalen Farbstofftransportfunktionen haben sich logarithmische Normalverteilungen bewährt. Im Falle der transcerebralen Doppelindikatorverdünnung kann daher für g(t) folgender Ansatz gewählt werden:

$$(6) \qquad g_{IGC}(t) = g_{\log nor}(t, mtt, \sigma) = \frac{1}{\sqrt{2\pi}\sigma t} \cdot e^{-\frac{(\ln\frac{t}{mtt}+\frac{\sigma^2}{2})^2}{2\sigma^2}}$$

In der hier angegebenen Form, die von der üblichen Schreibweise der logarithmischen Normalverteilung etwas abweicht, ist die Funktion so dargestellt, daß die mittlere Transitzeit mtt und die relative Dispersion $\sigma$ direkt die Parameter der Verteilung bilden.

Gegebenenfalls bestehen zwei unterschiedlich schnell durchblutete intravaskuläre Kompartimente. In diesem Fall kann die Transportfunktion durch die Summe von zwei Normalverteilungen repräsentiert werden, wobei $\alpha$ den Aufteilungsfaktor bildet:

$$(7) \qquad g_{IGC}(t) = \alpha_{ICG} \cdot g_{\log nor\, 1}(t, mtt_1, \sigma_1) +$$

$$+ (1 - \alpha_{ICG)} \cdot g_{\log nor\, 2}(t, mtt_2, \sigma_2)$$

Der Transportprozeß für die Indikatorkomponente Wärme oder Kälte kann in mehrere Abschnitte unterteilt werden:

1. Antransport überwiegend intravaskulär durch das arterielle Gefäßsystem, 2. Äquilibrierung des "hochdiffusiblen" Indikators Wärme oder Kälte auf der Ebene der Mikrozirkulation, 3. Abtransport der Wärme oder Kälte über das venöse Gefäßsystem. Der Transportprozeß für jeden Abschnitt läßt sich durch jeweils eine Transportfunktion beschreiben $g_{art}$(t), $g_{mikro}$(t), $g_{ven}$(t), der gesamte Transportprozeß resultiert dann aus der Faltung der drei Transportfunktionen:

$$(8) \qquad g_{therm}(t) = g_{art}(t) * g_{mikro}(t) * g_{ven}(t)$$

wobei der Operator "*" dem Faltungsintegral entspricht. Da die Faltung kommutativ ist, können $g_{art}$(t) und $g_{ven}$(t) zusammengefaßt werden als intravasale Transportfunktion. Diese wiederum, ist jedoch identisch mit der intravasalen Transportfunktion, die durch Entfaltung der Farbstoffkurven ermittelt werden kann. Es gilt also:

$$(9) \qquad g_{therm}(t) = g_{ICG}(t) * g_{mikro}(t)$$

Hierdurch läßt sich die Bestimmung von $g_{therm}$(t) auch bei eingeschränktem Signal/Rausch Verhältnis günstiger gestalten, da bei bekanntem $g_{ICG}$(t) durch Entfaltung der Farbstoffkurven lediglich die Parameter von $g_{mikro}$(t) ermittelt werden müssen. Die Äquilibrierung auf der Ebene der Mikrozirkulation ist als nahezu idealer Mischprozeß anzusehen. Für die Transportfunktion ist daher ein exponentieller Verlauf anzusetzen:

$$(10) \qquad g_{mikro}(t) = mtt_{mikro}\, e^{-\frac{t}{mtt_{mikro}}}$$

In der Regel wurde beobachtet, daß beim Gehirn durch die graue und weiße Substanz zwei unterschiedlich schnell durchblutete Kompartimente auftreten, deren Gesamttransportfunktion dann als Summe von zwei Exponential-Funktionen darstellbar ist, wobei $\alpha$ wiederum der Aufteilungfaktor ist:

$$(11) \qquad g_{mikro}(t) = \alpha \cdot mtt_{mikro\, 1}\, e^{-\frac{t}{mtt_{mikro\, 1}}} +$$

$$+ (1 - \alpha) \cdot mtt_{mikro\, 2}\, e^{-\frac{t}{mtt_{mikro\, 2}}}$$

Nachfolgend wird die Vorrichtung anhand der Zeichnung erläutert. In dieser zeigen:

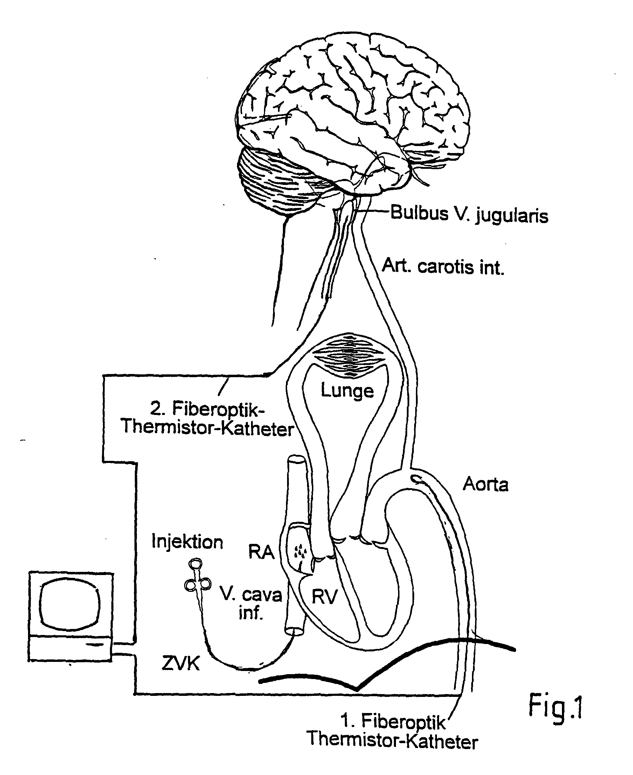

Fig. 1   Eine Messvorrichtung mit einem Schema zur Katheteranordnung,

Fig. 2   eine Darstellung einer typischen arteriellen und zerebralvenösen Farbstoffverdünnungskurve und

Fig. 3   eine Darstellung einer typischen arteriellen und zerebralvenösen Thermodilutionskurve.

Die Meßvorrichtung umfaßt zwei Meßsensoren, die jeweils als kombinierte Fiberoptik-Thermistor-Katheter ausgebildet und mit einem Rechner zur Auswertung verbunden sind. Ein erster Fiberoptik-Thermistor Katheter wird in der Aorta placiert, in dem er über ein entsprechendes Einführungsbesteck in die arteria femoralis eingebracht wird. Ein zweiter Fiberoptik-Thermistor-Katheter wird im Bulbus venae jugularis placiert, in dem er diesen durch retrograde Katheterierung der vena jugularis interna erreicht.

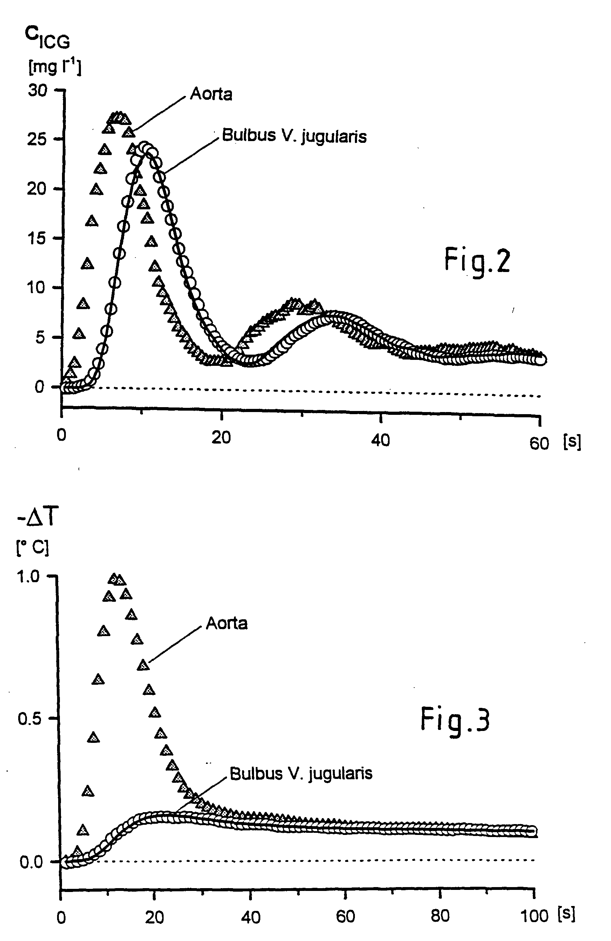

Nach venöser Bolusinjektion einer ausreichenden Indikatormenge, z. B. 0,2 - 0,4 mg / kg ICG in 0,3 - 0,5 ml / kg eisgekühlter Lösung, die z.B. über einen zentralen Venenkatheter ZVK erfolgt, werden die resultierenden Indikatorverdünnungskurven mittels der Fiberoptik-Thermistor-Katheter gemessen und durch den Rechner aufgezeichnet. Die Messung sollte über mindestens fünf Minuten durchgeführt werden. Bei der Messung ergeben sich die in den Fig. 2 und 3 dargestellten Indikatorverdünnungskurven, und zwar die Farbstoffverdünnungkurven gemäß Fig. 2, die eine typische arterielle (Dreiecke) und zerebralvenöse (leere Kreise) Farbstoffverdünnungskurve nach Injektion eines kalten Farbstoffbolus (25 mg) in den rechten Vorhof zeigt, und die Thermodilutionskurven gemäß Fig. 3, die eine typische arterielle (Dreiecke) und zerebralvenöse (leere Kreise) Thermodilutionskurve nach Injektion eines kalten Farbstoffbolus (0,4 ml / kg 3°C) in den rechten Vorhof zeigt.

Bei der Auswertung der Indikatorverdünnungskurven erfolgt zunächst eine Berechnung der intravasalen Transportfunktion $g_{ICG}(t)$ aus den Farbstoffkurven. Dazu werden die Werte der aufgezeichneten arteriellen a(t) und cerebralvenösen v(t) Farbstoffverdünnungskurve gemäß Fig. 2 in Gleichung (5) eingesetzt. Für g(t) wird als Modellfunktion der Ausdruck aus Gleichung (6) oder bei Berücksichtigung zweier Kompartimente der modifizierte Ausdruck aus Gleichung (7) eingesetzt. Nun werden die Parameter für die mittlere Transitzeit mtt und die relative Dispersion $\sigma$ solange variiert, bis die Differenz zwischen der gemessenen cerebralvenösen Indikatorkurve v(t) und dem Faltungsergebnis der gemessenen arteriellen Indikatorkurve a(t) nach Gleichung (5) minimiert. Danach stehen die mittlere Transitzeit $mtt_{ICG}$ und die Farbstofftransportfunktion $g_{ICG}(t)$ fest. Die relative Dispersion $\sigma$ wird hier nicht weiter ausgewertet.

Wird die arterielle Farbstoffkurve nach Fig. 2 mit der so erhaltenen Farbstofftransportfunktion $g_{ICG}(t)$ gefaltet, ergibt sich eine sehr genau an die zerebralvenöse Farbstoffkurve nach Fig. 2 angepaßte Kurve, wie sie durch die durchgezogene Linie dargestellt ist.

Anschließend erfolgt eine Berechnung der diffusiblen Transportfunktion $g_{therm}(t)$ aus den Thermodilutionskurven. Dazu werden die Werte der aufgezeichneten arteriellen a(t) und cerebralvenösen v(t) Thermodilutionskurve gemäß Fig. 3 in Gleichung (5) eingesetzt. Für g(t) wird der Ausdruck aus Gleichung (9) eingesetzt, wobei $g_{ICG}(t)$ bereits aus der vorherigen Berechnung bekannt ist und für $g_{mikro}(t)$ als Modellfunktion der Ausdruck aus Gleichung (10) oder bei Berücksichtigung zweier Kompartimente der modifizierte Ausdruck aus Gleichung (11) eingesetzt wird. Nun wird der einzige Parameter für die mittlere Transitzeit $mtt_{therm}$ solange variiert, bis die Differenz zwischen der gemessenen cerebralvenösen Indikatorkurve v(t) und dem Faltungsergebnis der gemessenen arteriellen Indikatorkurve a(t) nach Gleichung (5) minimiert. Danach stehen auch die mittlere Transitzeit $mtt_{therm}$ und die thermische Transportfunktion $g_{therm}(t)$ fest.

Wird die arterielle Thermodilutionskurve nach Fig. 3 mit der so erhaltenen thermischen Transportfunktion $g_{therm}(t)$ gefaltet, ergibt sich eine sehr genau an die zerebralvenöse Thermodilutionskurve nach Fig. 3 angepaßte Kurve, wie sie durch die durchgezogene Linie dargestellt ist.

Zur Ermittlung der Hirndurchblutung und des intracraniellen Blutvolumens müssen die erhaltenen Werte für die Transitzeiten $mtt_{therm}$ und $mtt_{ICG}$ nur noch in die Gleichungen (1) und (2) eingesetzt werden.

**Patentansprüche**

**1.** Vorrichtung zur Ermittlung der Hirndurchblutung, als CBF = "cerebral blood flow" bezeichnet, und des intracraniellen Blutvolumens, als ICBV = "intracerebral blood volume" bezeichnet, nach Injektion einer vorgegebenen Menge eines Doppelindikators in den Blutkreislauf und arterielle und venöse Messung der im Blutkreislauf resultierenden Indikatorverdünnungskurven, wobei die Vorrichtung Meßsensoren aufweist, die aus kombinierten Fiberoptik-Thermistor-Kathetern zur gesonderten Messung der Verdünnungkurven einer intravasalen Indikatorkomponente aus Farbstoff, vorzugsweise Indocyaningrün ICG, und einer hochdiffusiblen Indikatorkomponente aus Kälte oder Wärme bestehen, von denen der eine Meßsensor in einer zentralen Arterie vor dem Gehirn und der andere Meßsensor in einer zentralen Vene hinter dem Gehirn anordnerbar ist, wobei die Meßsensoren mit einem Rechner verbunden sind, der so gesteuert ist, daß aus den Meßwerten der Verdünnungkurven die Hirndurchblutung CBF aus dem Quotienten aus einem Verteilungskoeffizienten $\lambda_{therm}$ der hochdiffusiblen Indikatorkomponente zwischen Blut und dem Gehirngewebe und der mittleren Transitzeit $mtt_{therm}$ der hochdiffusiblen Indikatorkomponente durch das Gehirn berechnet wird und daß das intracranielle Blutvolumen ICBV aus dem Produkt aus Hirndurchblutung CBF und der mittleren Transitzeit $mtt_{ICG}$ der intravasalen Indikatorkomponente ICG berechnet wird.

2. Vorrichtung nach Anspruch 1, wobei der Rechner die mittleren Transitzeiten $mtt_{ICG}$ und $mtt_{therm}$ aus den transcerebralen Transportfunktionen der beiden Indikatoren $g_{therm}(t)$ und $g_{IGC}(t)$ bestimmt, wobei die mittleren Transitzeiten dem ersten Moment der Transportfunktionen gemäß der Beziehung

$$mtt \; = \; \frac{\int\limits_{0}^{\infty} g(t)\cdot t\; dt}{\int\limits_{0}^{\infty} g(t)\; dt}$$

entsprechen und die Transportfunktionen g(t) durch Entfaltung der gemessenen arteriellen a(t) und cerebralvenösen v(t) Indikatorkurven des Faltungsintegrals

$$v(t) \; = \; \int\limits_{0}^{\infty} g(t-u)a(u)du$$

ermittelt werden.

3. Vorrichtung nach Anspruch 2, wobei der Rechner die Transportfunktionen g(t) aus den gemessenen arteriellen a(t) und cerebralvenösen v(t) Indikatorkurven mit Hilfe eines iterativen, nichtlinearen Anpassungsverfahrens ermittelt, in dem unter Vorgabe einer den grundsätzlichen Verlauf der Transportfunktionen g(t) beschreibenden Modellfunktion die Parameter der Modellfunktion nach dem Verfahren der kleinsten Quadrate variiert werden, bis die Differenz zwischen der gemessenen cerebralvenösen Indikatorkurve v(t) und dem Faltungsergebnis der gemessenen arteriellen Indikatorkurve a(t) nach der Beziehung

$$\left[ v(t) - \int\limits_{0}^{\infty} g(t-u)\, a(u)\; du \right]^2 \; = \; \min$$

minimiert.

4. Vorrichtung nach Anspruch 3, wobei als Modellfunktion für die intravasale Farbstofftransportfunktionen $g_{ICG}(t)$ eine logarithmische Normalverteilung der Form

$$g_{IGC}(t) = g_{\log\,nor}(t,mtt,\sigma) = \frac{1}{\sqrt{2\pi}\sigma t} \cdot e^{-\frac{(\ln\frac{t}{mtt}+\frac{\sigma^2}{2})^2}{2\sigma^2}}$$

gewählt wird, wobei die zu variierenden Parameter durch die mittlere Transitzeit mtt der Farbstofflösung ICG und durch die relativer Dispersion σ gebildet werden.

**5.** Vorrichtung nach Anspruch 4, wobei die Modellfunktion für zwei unterschiedlich stark durchblutete Kompartimente durch die Summe von zwei logarithmischen Normalverteilungen der Form

$$g_{IGC}(t) = \alpha_{ICG} \cdot g_{\log nor\ 1}\ (t, mtt_1, \sigma_1) +$$

$$+ (1 - \alpha_{ICG}) \cdot g_{\log nor\ 2}\ (t, mtt_2, \sigma_2)$$

modifiziert wird.

**6.** Vorrichtung nach Anspruch 4, wobei die Transportfunktion $g_{therm}(t)$ der hochdiffusiblen Indikatorkomponente durch Faltung der zuvor bestimmten Transportfunktion $g_{ICG}(t)$ der intravasalen Indikatorkomponente ICG mit einem eine Mikrozirkulation beschreibenden Anteil $g_{mikro}(t)$ des Transportprozesses der hochdiffusiblen Indikatorkomponente in der Form

$$g_{therm}\ (t) = g_{ICG}\ (t) * g_{mikro}\ (t)$$

gefaltet wird, wobei der Operator "*" dem Faltungsintegral entspricht, und daß für die Transportfunktion der Mikrozirkulation als Modellfunktion eine Exponentialfunktion der Form

$$g_{mikro}(t) = mtt_{mikro}\ e^{-\frac{t}{mtt_{mikro}}}$$

gewählt wird, in der die mittlere Transitzeit $mtt_{mikro}$ der Mikrozirkulation als variabler Parameter enthalten ist.

**7.** Vorrichtung nach Anspruch 6, wobei die Modellfunktion für zwei unterschiedlich stark durchblutete Kompartimente durch die Summe von zwei Exponentialfunktionen der Form

$$g_{mikro}(t) = \alpha_{mikro} \cdot mtt_{mikro\ 1}\ e^{-\frac{t}{mtt_{mikro\ 1}}} +$$

$$+ (1 - \alpha_{mikro}) \cdot mtt_{mikro\ 2}\ e^{-\frac{t}{mtt_{mikro\ 2}}}$$

modifiziert wird.

## Claims

**1.** Device for determining the cerebral blood flow or CBF and the intracerebral blood volume or ICBV, following the injection of a predetermined quantity of a double indicator into the blood stream and arterial and venous measurement of the resulting indicator dilution curves in the blood stream, the device having measuring sensors comprising combined fibre optic thermistor catheters for the separate measurement of the dilution curves of an intravascular indicator component of dye, preferably indocyanine dye ICG, and a highly diffusible indicator component of cold or heat, whereof one measuring sensor can be placed in a central artery in front of the brain and the other measuring sensor in a central vein behind the brain, the measuring sensors being connected to a computer, which is controlled in such a way that from the measured values of the dilution curves the cerebral blood flow CBF is calculated from the quotient of a distribution coefficient $\lambda_{therm}$ of the highly diffusible indicator component between blood and the brain tissue and the average transit time $mtt_{therm}$ of the highly diffusible indicator component through the brain and that the intracerebral blood volume ICBV is calculated from the product of the cerebral blood flow CBF and the average transmit time $mtt_{ICG}$ of the intravascular indicator component ICG.

**2.** Device according to claim 1, wherein the computer determines the average transit times $mtt_{ICG}$ and $mtt_{therm}$ from

the transcerebral transport functions of the two indicators $g_{therm}(t)$ and $g_{IGC}(t)$, the average transit times corresponding to the first moment of the transport functions in accordance with the relationship

$$mtt = \frac{\int_0^{\infty} g(t) \cdot t \ dt}{\int_0^{\infty} g(t) \ dt}$$

and the transport functions g(t) are determined by the deconvolution of the measured arterial a(t) and cerebral venous v(t) indicator curves of the convolution integral

$$v(t) = \int_0^{\infty} g(t-u) a(u) du$$

3. Device according to claim 2, wherein the computer determines the transport functions g(t) from the measured arterial a(t) and cerebral venous v(t) indicator curves with the aid of an iterative, non-linear adaptation process, in which accompanied by the presetting of a model function describing the fundamental course of the transport functions g(t) the parameters of the model function are varied in accordance with the method of least squares until the difference between the measured cerebral venous indicator curve v(t) and the convolution result of the measured arterial indicator curve a(t) is minimized in accordance with the relationship

$$\left[ v(t) - \int_0^{\infty} g(t-u) a(u) du \right]^2 = min$$

4. Device according to claim 3, wherein as the model function for the intravascular dye transport functions $g_{ICG}(t)$ is selected a logarithmic normal distribution of form

$$g_{ICG}(t) = g_{log\ nor}(t, mtt, \sigma) = \frac{1}{\sqrt{2\pi}\sigma t} \cdot e^{-\frac{(\ln\frac{t}{mtt} + \frac{\sigma^2}{2})^2}{2\sigma^2}}$$

in which the parameters to be varied are formed by the average transit time mtt of the dye solution ICG and by the relative dispersion $\sigma$.

5. Device according to claim 4, wherein the model function for two compartments having a blood flow of greatly differing intensity is modified by the sum of two logarithmic normal distributions of form

$$g_{ICG}(t) = \alpha_{ICG} \cdot g_{log\ nor\ 1}(t, mtt_1, \sigma_1) +$$

$$+ (1 - \alpha_{ICG}) \cdot g_{log\ nor\ 2}(t, mtt_2, \sigma_2)$$

6. Device according to claim 4, wherein the transport function $g_{therm}(t)$ of the highly diffusible indicator component is convoluted by convoluting the previously determined transport function $g_{ICG}(t)$ of the intravascular indicator component ICG with a fraction $g_{micro}(t)$ of the transport process of the highly diffusible indicator component describing a microcirculation in the form

$$g_{therm}(t) = g_{ICG}(t) * g_{micro}(t)$$

in which the operator * corresponds to the convolution integral, and wherein for the transport function of the microcirculation as the model function is chosen an exponential function of form

$$g_{micro}(t) = mtt_{micro} \; e^{-\frac{t}{mtt_{micro}}}$$

in which the average transit time $mitt_{micro}$ of the microcirculation is contained as a variable parameter.

7. Device according to claim 6, wherein the model function is modified for two compartments with a greatly differing blood flow by the sum of two exponential functions of form

$$g_{micro}(t) = \alpha_{micro} \cdot mtt_{micro\,1} \; e^{-\frac{t}{mtt_{micro\,1}}} +$$

$$+ (1-\alpha_{micro}) \cdot mtt_{micro\,2} \; e^{-\frac{t}{mtt_{micro\,2}}}.$$

## Revendications

1. Dispositif de détermination du débit sanguin cérébral, désigné par CBF = cerebral blood flow, et du volume sanguin intracérébral, désigné par ICBV = intracerebral blood volume, dans lequel, après avoir injecté d'une quantité prédéterminée d'un double indicateur dans la circulation sanguine et avoir fait des mesures artérielles et veineuses, on obtient des courbes de dilution d'indicateur dans la circulation sanguine, le dispositif comportant des capteurs de mesure composés de cathéters, avec thermistor et fibre optique, pour des mesures séparées des courbes de dilution d'une composante indicatrice intravasculaire de colorant, de préférence du vert d'indocyanine ICG, et d'une composante indicatrice à haute diffusion de froid ou de chaleur, parmi lesquels le premier capteur de mesure peut être disposé dans une artère centrale en amont du cerveau et l'autre dans une veine centrale derrière le cerveau, les capteurs de mesure étant reliés à un calculateur commandé de façon à calculer à partir des valeurs de mesure des courbes de dilution, le débit sanguin cérébral CBF sur la base du quotient d'un coefficient de distribution $\lambda_{therm}$ de la composante indicatrice de haute diffusion entre sang et tissu cérébral et du temps de transit moyen $mtt_{therm}$ de la composante indicatrice de haute diffusion dans le cerveau et qu'on calcule le volume sanguin intracérébral ICBV sur la base du produit du débit sanguin cérébral CBF et du temps de transit moyen $mtt_{therm}$ de la composante indicatrice intravasculaire ICG.

2. Dispositif suivant la revendication 1, dans lequel le calculateur détermine les temps de transit moyens $mtt_{ICG}$ et $mtt_{therm}$ sur la base des fonctions de transport transcérébrales des deux indicateurs $g_{therm}$ (t) et $g_{IGC}$ (t), les temps de transit moyens correspondant au premier moment des fonctions de transport suivant la relation

$$mtt \; = \; \frac{\int_{0}^{\infty} g(t) \cdot t \; dt}{\int_{0}^{\infty} g(t) \; dt}$$

et les fonctions de transport g(t) étant obtenues par le développement des courbes indicatrices mesurées artérielles a(t) et veineuses cérébrales v(t) de l'intégrale de convolution

$$v(t) \; = \; \int_{0}^{\infty} g(t-u)a(u)du$$

**3.** Dispositif suivant la revendication 2, dans lequel le calculateur détermine les fonctions de transport g(t) à partir des courbes indicatrices mesurées artérielles a(t) et veineuses cérébrales v(t) à l'aide d'un procédé d'adaptation itératif et non linéaire, dans lequel, en prenant comme base une fonction modèle décrivant le tracé fondamental des fonctions de transport g(t), on fait varier les paramètres de la fonction modèle en suivant le procédé des moindres carrés, jusqu'à ce que la différence entre la courbe indicatrice de la veine cérébrale mesurée v(t) et le résultat du développement de la courbe indicatrice artérielle mesurée a(t) soit minimale suivant la relation

$$\left[ v(t) - \int_0^\infty g(t-u)\,a(u)\,du \right]^2 = \min$$

**4.** Dispositif suivant la revendication 3, dans lequel, comme fonction modèle pour la fonction de transport de colorant intravasculaire $g_{ICG}(t)$, on choisit une distribution log-normale de forme

$$g_{IGC}(t) = g_{\log\,nor}(t, mtt, \sigma) = \frac{1}{\sqrt{2\pi}\sigma t} \cdot e^{-\frac{(\ln\frac{t}{mtt}+\frac{\sigma^2}{2})^2}{2\sigma^2}}$$

les paramètres à faire varier étant formés par le temps de transit moyen mtt de solution du colorant ICG et la dispersion relative $\sigma$.

**5.** Dispositif suivant la revendication 4, dans lequel on modifie la fonction modèle pour deux compartiments sanguins très différents par la somme de deux distributions log-normales de forme

$$g_{IGC}(t) = \alpha_{ICG} \cdot g_{\log\,nor\,1}(t, mtt_1, \sigma_1) +$$

$$+ (1 - \alpha_{ICG}) \cdot g_{\log\,nor\,2}(t, mtt_2, \sigma_2)$$

**6.** Dispositif suivant la revendication 4, dans lequel on sépare la fonction de transport $g_{therm}(t)$ de la composante indicatrice à haute diffusion par convolution de la fonction de transport déterminée d'avance $g_{ICG}(t)$ de la composante indicatrice intravasculaire ICG avec une portion décrivant une microcirculation $g_{micro}(t)$ du procédé de transport de la composante indicatrice à haute diffusion dans la forme

$$g_{therm}(t) = g_{ICG}(t) * g_{micro}(t)$$

l'opérateur « $*$ » correspondant à l'intégrale de convolution, et on choisit, comme fonction modèle pour la fonction de transport de la microcirculation, une fonction exponentielle de la forme

$$g_{mikro}(t) = mtt_{mikro}\,e^{-\frac{t}{mtt_{mikro}}}$$

dans laquelle le temps de transit moyen $mtt_{micro}$ de la microcirculation est pris comme paramètre variable.

**7.** Dispositif suivant la revendication 6, dans lequel on modifie la fonction modèle pour deux compartiments sanguins très différents par la somme de deux distributions log-normales de forme

$$g_{mikro}(t) = \alpha_{mikro} \cdot mtt_{mikro\,1}\,e^{-\frac{t}{mtt_{mikro\,1}}} +$$

$$+ (1 - \alpha_{mikro}) \cdot mtt_{mikro\ 2}\ e^{-\frac{t}{mtt_{mikro\ 2}}}$$

Fig.1

Fig.2

Fig.3